# EUROPÄISCHE PATENTANMELDUNG

(11) **EP 1 695 672 A1**
(43) Veröffentlichungstag der Anmeldung: **30.08.2006**
(21) Anmeldenummer: 06002710.9
(22) Anmeldetag: 09.02.2006
(51) Int. Cl.: A61C 9/00, A61C 19/06, A61F 13/36

(54) **Kompressenkappe für Zahnstümpfe und Verfahren zum Trocknen eines Zahnstumpfes**

(30) Priorität: 23.02.2005 DE 102005008355
(71) Anmelder: Coltène/Whaledent GmbH + Co. KG, 89129 Langenau (DE)
(72) Erfinder: Mannschedel, Werner, 89129 Langenau (DE); Müller, Barbara, 89129 Langenau (DE)
(74) Vertreter: Forstmeyer, Dietmar

(57) **Zusammenfassung**

Die vorliegende Erfindung betrifft eine Kompressenkappe (1) für Zahnstümpfe (5) und ein Verfahren zum Trocknen eines Zahnstumpfes (5). Die Kompressenkappe (1) für Zahnstümpfe (5) besitzt einen Hohlraum (7) zur Aufnahme einer einen Zahnstumpf (5) umgebenden saugfähigen Schaumstoffmasse (8). Der Hohlraum (7) hat eine einseitige Öffnung (9) zur Aufnahme der Reagenzien (10, 11) für die Schaumstoffmasse (8) und zur Aufnahme des Zahnstumpfes (5) und ist von Wänden umgeben, deren Wandstärke (d) zur Öffnung (9) hin vermindert ist. Der Öffnungsrand (14) verfügt über ein derartiges räumliches Profil (15), dass der Öffnungsrand (14) eine Dichtung (16) im angepressten Zustand der Kompressenkappe (1) an die Interdentalpapille (17) eines Zahnstumpfes (5) im Bereich der Gingiva-Tasche (18) für den Sulcus (19) darstellt.

## Beschreibung

Die vorliegende Erfindung betrifft eine Kompressenkappe für Zahnstümpfe, die ein verformbares und gegebenenfalls für Körperflüssigkeiten saugfähiges Material aufweist. Dazu besitzt die Kompressenkappe einen Hohlraum zur Aufnahme eines Zahnstumpfes.

Derartige Kompressenkappen sind unter dem Handelsnamen "Comprecap" bekannt. Diese Kompressen dienen der technischen Vorbereitung einer Abdrucknahme von Zahnstümpfen. Vor einer derartigen Abdrucknahme sind die Oberflächen des Zahnstumpfes und seiner Umgebung, insbesondere die Gingiva-Tasche mit ihrem Sulcus, von Körperflüssigkeit zu befreien und zu trocknen. Dazu wird bisher ein Retraktionsfaden, der eine adstringierende, blutstillende Lösung aus 10%-igem AluminiumChlorid-Hexahydrat enthält, um den Zahn gewickelt und in die Gingiva-Tasche eingepresst. Das blutstillende Mittel des getränkten Refraktionsfadens verursacht keine Kreislaufbeeinträchtigungen und dient der Vorbehandlung der Gingiva. Mit einem derartigen Retraktionsfaden wird der Sulcus der Gingiva-Tasche geöffnet und überschüssige Körperflüssigkeit, wie Blut und Speichel, abgesaugt.

Die "Comprecap" dient als technisches Hilfsmittel dazu, möglichst eng anliegend an dem Zahnstumpf geführt zu werden, um den Retraktionsfaden möglichst tief in den Sulcus der Gingiva-Tasche einzupressen und die Gingiva-Tasche zu weiten. Gleichzeitig dient die Kompressenkappe der Trocknung der Oberflächen und dem Absaugen von Körperflüssigkeiten in der Umgebung des Zahnstumpfes. Im Prinzip dient die Kompressenkappe dazu, einen Faden, der vorher um den Zahnstumpf gewikkelt wurde, bis in den Schmelz-Zement-Grenzbereich des Zahnstumpfes vorzuschieben. Dazu ist es erforderlich, dass sich die Kompressenkappe möglichst eng an den Zahnstumpf anschmiegt, um den Retraktionsfaden bis in den Schmelz-Zement-Grenzbereich um den Zahn vorzutreiben.

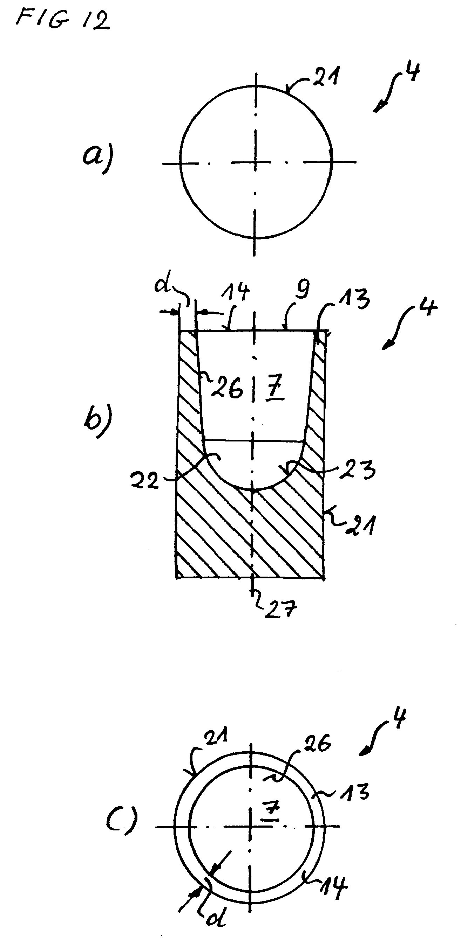

Ein derartiges Hilfsmittel in Form einer Kompressenkappe 4 zeigt Figur 12, wobei in Figur 12 a) eine Druntersicht der Kompressenkappe 4 dargestellt wird, in Figur 12 b) ein Längsquerschnitt durch die Kompressenkappe 4 abgebildet ist und in Figur 12 c) eine Draufsicht auf die Kompressenkappe 4 gezeigt wird. Derartige Kompressenkappen 4 in Form der so genannten "Comprecap" sind in unterschiedlichen Größen verfügbar, sodass sie an die unterschiedlichen Zahnvolumina der Zahnstümpfe anpassbar sind. Die Druntersicht in Figur 12 a) zeigt, dass die Kompressenkappen zylindrisch sind und Figur 12 b) zeigt, dass die Kompressenkappen einen Hohlraum 7 aufweisen, der eine halbkreisförmige Innenkontur 23 im Bodenbereich 22 hat und eine leicht konische Innenwand 26 besitzt, die sich zum Öffnungsrand 14 hin in ihrer Wandstärke vermindert. Der Öffnungsrand 14 weist ein glattes Profil auf, sodass ein gleichmäßiger Andruck über dem Umfang der Öffnungsfläche 14 auf den Retraktionsfaden möglich ist. In Figur 12 c) ist zu sehen, dass der Öffnungsrand 14 der Kompressenkappe 4 gemäß dem Stand der Technik scheibenförmig ist, wobei die Wandstärke in dem Öffnungsrandbereich 14 d ist, die derart bemessen ist, dass der Öffnungsrand in den Sulcus der Gingiva-Tasche einführbar ist.

Diese Kompressenkappe hat den Nachteil, dass sie lediglich als technisches Werkzeug einen Druck auf einen Retraktionsfaden ausübt, und dabei den Sulcus vor einem Abdruck des Zahnstumpfes so weit wie möglich öffnet. Dabei zeigt es sich, dass sowohl das Verfahren der,Anbringung des Fadens, als auch des Einbringens der Kompressenkappe in den Sulcus eine äu-βerst schmerzhafte Vorbereitung für einen Abdruck eines Zahnstumpfes darstellt. Auch hängt es stark von der Geschicklichkeit der behandelnden Person beim Einführen des Retraktionsfadens und beim Eindrücken des Öffnungsrandes in den Sulcus ab, in wieweit der Sulcus selbst von den Körperflüssigkeiten befreit wird. Somit ist sowohl die Kompressenkappe als solche als auch das Trocknungsverfahren von Zahnstümpfen und ihrer Umgebung mit erheblichen Nachteilen belastet.

Demnach besteht ein Bedarf, eine gründlichere Absaugung von Körperflüssigkeiten in der Umgebung und auf einem Zahnstumpf zu ermöglichen und zum anderen besteht der Bedarf, dafür geeigneter Kompressenkappen zu schaffen.

Aufgabe der Erfindung ist es, einerseits ein neuartiges Verfahren zur Vorbereitung von Abdrücken von Zahnstümpfen anzugeben und eine Kompressenkappe zu schaffen, die gegenüber den bisher verfügbaren Kompressenkappen ein verbessertes Design aufweist und funktionsgerecht für das neue Verfahren konstruiert ist, sodass mit geringerem Zeitaufwand und verbessertem Werkzeug in Form einer neuartigen Kompressenkappe ein Trokkenlegen von Zahnstümpfen und umgebenden Gingiva-Taschen ermöglicht wird.

Gelöst wird diese Aufgabe mit dem Gegenstand der unabhängigen Ansprüche. Vorteilhafte Weiterbildungen der Erfindung ergeben sich aus den abhängigen Ansprüchen.

Erfindungsgemäß wird eine Kompressenkappe für Zahnstümpfe geschaffen, die ein bei Raumtemperatur verformbares Material aufweist. Die erfindungsgemäße Kompressenkappe besitzt einen Hohlraum zur Aufnahme einer einen Zahnstumpf umgebenden saugfähigen und expandierenden Kunststoffmasse. Der Hohlraum hat eine einseitige Öffnung zur Aufnahme der Reagenzien für die expandierende Schaumstoffmasse und zur Aufnahme des Zahnstumpfes und ist von Wänden umgeben, deren Wandstärke zur Öffnung hin vermindert ist. Der Öffnungsrand verfügt über ein derartiges räumliches Profil, dass der Öffnungsrand eine Dichtung im angepressten Zustand der Kompressenkappe an die Interdentalpapille eines Zahnstumpfes im Bereich der Gingiva-Tasche für den Sulcus darstellt.

Diese Kompressenkappe hat einerseits den Vorteil, dass sie nicht mit ihrem Öffnungsrand in den Sulcus um den Zahnstumpf herum eindringt. Vielmehr dichtet sie mit ihrem profilierten Öffnungsrand den Bereich um den Zahnstumpf herum ab. Somit ist es möglich, ein vollständig neues Verfahren mit dieser Kompressenkappe zum Abtrocknen und Präparieren von Zahnstümpfen durchzuführen. Dabei ist es nicht mehr nötig, mit entsprechenden Spreizwerkzeugen einen Retraktionsfaden um den Zahn in dem Sulcus zu legen, um diesen aufzuspreizen. Auch ist es nicht mehr nötig, einen relativ stumpfen planaren Umfangsrand einer Kompressenkappe in den Sulcus nachzuschieben, es kann vielmehr der profilierte Öffnungsrand nun verwendet werden, um die Gingiva-Tasche abzudichten, um den Sulcus durch einen expandierenden saugfähigen Schaumstoff zu spreizen und von Körperflüssigkeiten zu befreien. Die Funktionsweise und die Konstruktion der erfindungsgemäßen Kompressenkappe unterscheidet sich somit deutlich vom bisherigen Stand der Technik und ermöglicht eine verbesserte und intensivere Trockenlegung eines Zahnstumpfes und seiner Umgebung z.B. in Vorbereitung eines Abdruckverfahrens.

Vorzugsweise weist die Kompressenkappe als verformbares Material ein elastomeres Material auf. Dieses elastomere Material legt sich abdichtend an die Interdentalpapille an und dichtet den Hohlraum der Kompressenkappe gegenüber der Mundhöhle wirkungsvoll ab. Als elastomeres Material kommt z.B. ein Silikon zum Einsatz.

In einer bevorzugten Ausführungsform der Kompressenkappe weist diese als verformbares Material ein Material auf, das gleichzeitig saugfähig ist. Vorzugsweise weist dazu die Kompressenkappe eine für Körperflüssigkeiten saugfähige gepresste Watte und/oder einen für Körperflüssigkeiten saugfähigen Filz und/oder einen für Körperflüssigkeiten saugfähigen Schaumstoff auf. Diese Materialien haben den Vorteil, dass sie im Bereich des profilierten Öffnungsrandes ausreichend nachgiebig sind, um sich dichtend an die Interdentalpapille der Gingiva-Tasche anzulegen. Außerdem sorgt durch dieses Anlegen die Kompressenkappe für eine Abdichtung des Hohlraums gegenüber der Umgebung, sodass sich die aus den Reagenzien bildende und expandierende Schaumstoffmasse um den Zahnstumpf und bis tief hinein in den Sulcus ausdehnen kann.

Für eine vorteilhafte Ausführungsform der Erfindung weist die Kompressenkappe als verformbares Material ein elastisches Material auf, das plastisch verformbar ist. Ein derartiges Material hat den Vorteil, dass es anpassungsfähig und nachgiebig ist und sich an die Interdentalpapille anschmiegt und für eine gute Abdichtung sorgt.

In einer weiteren bevorzugten Ausführungsform der Erfindung hat die Kompressenkappe eine zylindrische Außenkontur. Diese zylindrische Außenkontur ermöglicht es, beim Abziehen der Kompressenkappe diese zu drehen. Dabei wird vorzugsweise auch die saugfähige und expandierte Schaumstoffmasse, die aufgrund der Expansion des Schaumstoffes und gegebenenfalls auch aufgrund der Eigenschaften des Materials der Kompressenkappe in dieses Material einfiltriert ist, mit gedreht und lässt sich auf einfache Weise von dem Zahnstumpf abziehen.

In einer weiteren bevorzugten Ausführungsform weist die Kompressenkappe einen elliptischen Querschnitt auf. Dieser Querschnitt kann für besonders geformte Zähne vorgesehen werden, hat jedoch das Problem, dass ein Drehen dieser Kappe zum Ablösen des Schaumstoffes von der Oberfläche des Zahnstumpfes behindert wird. Gemäß einer weiteren Ausführungsform können die erfindungsgemäßen Kompressenkappen zumindest an zwei einander gegenüberliegenden Seiten flache Flächen aufweisen, so dass mehrere Kompressenkappen nebeneinander zum Einsatz kommen können. Weiterhin kann die Kompressenkappe einen rechtekkigen Querschnitt aufweisen, und einen Außenmantel besitzen, der glatte Flächen aufweist. Das hat den Vorteil, dass sich die Kompressenkappe hervorragend handhaben lässt und in der Präparationsphase sicher positioniert werden kann, um beispielsweise die Ausgangsprodukte für die Schamstoffmasse zuverlässig in den Hohlraum einfüllen zu können.

In einer weiteren Ausführungsform der Erfindung weist der Hohlraum im Bodenbereich eine halbkugelförmige Innenkontur auf. Eine derartige Innenkontur hat den Vorteil, dass diese Halbkugel bereits ein erstes Reagenz der saugfähigen, expanierenden Schaumstoffmasse in Form eines Gels oder in einer anderen Konsistenz aufnehmen kann.

In einer weiteren Ausführungsform der Erfindung weisen die Wände eine zylindrische Innenkontur auf. Eine derartige zylindrische Innenkontur hat den Vorteil, dass die Wandstärke der Kompressenkappe minimiert werden kann und somit äußerst klein und dünn gestaltet werden kann.

In einer weiteren bevorzugten Ausführungsform der Erfindung weist die Kompressionskappe Wände auf, welche eine parabolische Innenkontur besitzen. Auch bei der parabolischen Innenkontur ist es möglich, im Bodenbereich des Hohlraums bereits ein erstes Reagenz zu deponieren, das dann für die Reaktion mit einem zweiten Reagenz zu einem expandierenden Schaumstoff zur Verfügung steht.

Weiterhin ist es möglich, dass die Wände eine konische Innenkontur aufweisen. An diese konische Innenkontur kann sich wiederum ein halbkugelförmiger oder ein kugelkappenförmiger Bodenbereich zur Aufnahme eines Reagenz anschließen.

Weiterhin ist es möglich, dass die Wände eine elliptische Innenkontur aufweisen, wobei die Innenkontur lediglich eine Halbellipse darstellt, sodass sich zum Öffnungsrand hin die Wandstärke der Kompressenkappe vermindert.

Vorzugsweise weisen die Wände (13) eine gestufte Innenkontur auf. Die Stufen der Innenkontur können dazu Abschnitte der zylindrischen, der parabolischen, der elliptische oder der konischen Innenkontur in Stufen aufweisen. Diese Ausführungsform hat den Vorteil, dass sie den Zahnstumpfformen eng anpassbar ist.

Weiterhin ist es möglich, dass die Wandstärke im Bereich der Öffnung einer Zahnschmelzdicke entspricht. Bei dieser Dimensionierung der Wandstärke der Kompressenkappe ist es vorteilhaft, wenn der Zahnstumpf von Zahnschmelz befreit wurde und der Zahnstumpf im Wesentlichen aus Dentin besteht.

Vorzugsweise ist der Öffnungsrand zwar scheibenförmig, wie bei herkömmlichen Kompressenkappen, jedoch ist das Profil dieser Scheibenform durch zwei gegenüber liegende Aussparungen deutlich verändert, die sich bei Anpressdruck auf die Interdentalpapille im Bereich einer Gingiva-Tasche zwischen den Zahnreihen anschmiegen. Diese Aussparungen des Öffnungsrandes weisen als räumliches Profil vorzugsweise einen Kreisausschnitt oder einen Halbkreis auf. Somit können die Bereiche des räumlichen Profils, die keine Aussparung darstellen, weiter über das Zahnfleisch greifen als die Bereiche, die die Interdentalpapille zwischen den Zähnen abdecken. Damit wird die oben erwähnte Abdichtung in vorteilhafter Weise gewährleistet und sorgt dafür, dass nicht die Kompressenkappe in den Sulcus gepresst wird, sondern der expandierende Schaumstoff sich zunächst in dem Sulcus ausbreitet und diesen öffnet, um wirkungsvoll die gesamte Körperflüssigkeit der Gingiva-Tasche aufzusaugen und die Umgebung des Zahnstumpfes und den Zahnstumpf selbst trocken legt.

Außerdem ist es möglich, eine Kompressenkappe vorzusehen, die mehrere nebeneinanderliegende Hohlräume für mehrere nebeneinander angeordnete Zahnstümpfe für eine gleichzeitige Trocknung derselben aufweist. Eine derartige Kompressenkappe hat den Vorteil, dass sie sicher handhabbar ist, insbesondere bei der Trockenlegung mehrerer benachbarter Zahnstümpfe. Schließlich ist es auch möglich, eine Kompressenkappe vorzusehen, die eine Reihe nebeneinanderliegender Hohlräume für eine Vielzahl zu trocknender Zahnstümpfe aufweist, wobei zwischen den Hohlräumen Trennwände angeordnet sind und wobei zwischen den Trennwänden Trennfugen angeordnet sind, um Einzelkompressenkappen oder auch mehrere Kompressenkappen von der Vielzahl der in Reihe angeordneten Hohlräume abzutrennen, indem entlang der Trennfugen die gewünschte Abtrennung einer begrenzten Anzahl oder einzelner Kompressen durchgeführt wird.

Ein Verfahren zum Trocknen eines Zahnstumpfes und seiner Umgebung mittels einer Kompressenkappe gemäß den obigen Ausführungen weist die nachfolgenden Verfahrensschritte auf. Zunächst wird ein erstes Reagenz im Bodenbereich des Hohlraums der Kompressenkappe zum Bilden einer saugfähigen expandierenden Kunststoffmasse deponiert. Anschließend wird der Zahnstumpf mit einem zweiten Reagenz zum Bilden der saugfähigen Schaumstoffmasse benetzt. Danach wird die Kompressenkappe unter Einführen des Zahnstumpfes in den Hohlraum der Kompressenkappe angelegt. Dann wird der räumlich profilierte Öffnungsrand der Kompressionskappe an die Interdentalpapille des Zahnstumpfes im Bereich der Gingiva-Tasche zum Abdichten des Sulcus angepresst. Das kann vorzugsweise durch Schließen oder Aufeinanderpressen der Zahnreihen geschehen. Beim Eintauchen des mit dem zweiten Reagenz benetzten Zahnstumpfes in das erste Reagenz in dem Bodenbereich des Hohlraums der Kompressenkappe bildet sich die saugfähige und expandierende Schaumstoffmasse durch Reaktion der beiden Reagenzien aus. Dabei dringt die Schaumstoffmasse in den Sulcus der Gingiva-Tasche. Es erfolgt ein Aufsaugen der Körperflüssigkeiten durch die Schaumstoffmasse. Schließlich kann der Schaumstoff von den getrockneten Oberflächen der Gingiva-Tasche des Zahnstumpfes unter Abziehen der Kompressenkappe mit anhaftendem Schaumstoff entfernt werden.

Dieses Verfahren hat den Vorteil, dass es ohne große Schmerzen zu verursachen und in verkürzter Arbeitszeit durchgeführt werden kann. Das umständliche Umschlingen des Zahnstumpfes im Bereich des Sulcus und ein entsprechend schmerzhaftes Nachdrücken des Retraktionsfadens in den Sulcus hinein durch eine herkömmliche Kompressenkappe entfällt vollständig. Somit wird durch ein angenehmes Einpassen der neuartigen Kompressenkappe auf die Gingiva der Umgebung des Zahnstumpfes eine schnelle, sichere und den Sulcus schmerzfrei erweiternde Abtrocknung der Oberflächen und der Umgebung des Zahnstumpfes erreicht.

Ein weiteres bevorzugtes Verfahren zum Trocknen eines Zahnstumpfes und seiner Umgebung mittels einer Kompressenkappe weist die nachfolgenden Verfahrenschritte auf:
- Deponieren eines ersten Reagenz in einer ersten Kammer eines Mischers und Spenders;
- Deponieren eines zweiten Reagenz in einer zweiten Kammer eines Mischers und Spenders;
- Mischen der beiden Reagenzien in einer Mischkammer des Mischers;
- Spenden einer vorgesehenen Menge der gemischtren Reagenzien in eine Kompressenkappe und/oder auf einen Zahnstumpf;
- Anlegen der Kompressenkappe unter Einführen des Zahnstumpfes in den Hohlraum der Kompressenkappe;
- Anpressen des räumlich profilierten Öffnungsrandes der Kompressenkappe an die Interdentalpapille des Zahnstumpfes im Bereich der Gingiva-Tasche zum Abdichten des Sulcus;
- Bilden der saugfähigen Schaumstoffmasse aus den gemischten Reagenzien;
- Eindringen der Schaumstoffmasse in den Sulcus der Gingiva-Tasche;
- Aufsaugen der Körperflüssigkeiten durch die Schaumstoffmasse;
- Entfernen des Schaumstoffs von den getrockneten Oberflächen der Gingiva-Tasche und des Zahnstumpfes unter Abziehen der Kompressenkappe mit anhaftendem Schaumstoff.

Dieses Verfahren hat den Vorteil, dass durch den Einsatz des Mischers mit Spender, z.B. einer Doppelkammerspritze mit statischem Mischer, eine präzise Dosierung der zu mischenden Reagenzien erfolgen kann, bevor die Kompressionskappe mit der Mischung in Berührung kommt.

Ein weiteres alternatives Verfahren zum Trocknen eines Zahnstumpfes und seiner Umgebung mittels einer Kompressenkappe weist die nachfolgenden Verfahrenschritte auf:
- Deponieren eines kühl gelagerten Einkomponentenschaumstoffs in einen Spender;
- Spenden einer vorgesehenen Menge des Einkomponentenschaumstoffs in eine Kompressenkappe;
- Anlegen der Kompressenkappe unter Einführen des Zahnstumpfes in den Hohlraum der Kompressenkappe;
- Anpressen des räumlich profilierten Öffnungsrandes der Kompressenkappe an die Interdentalpapille des Zahnstumpfes im Bereich der Gingiva-Tasche zum Abdichten des Sulcus;
- Bilden der saugfähigen Schaumstoffmasse aus dem Einkomponentenschaumstoff;
- Eindringen der Schaumstoffmasse in den Sulcus der Gingiva-Tasche;
- Aufsaugen der Körperflüssigkeiten durch die Schaumstoffmasse;
- Entfernen des Schaumstoffs von den getrockneten Oberflächen der Gingiva-Tasche und des Zahnstumpfes unter Abziehen der Kompressenkappe mit anhaftendem Schaumstoff.

Dieses Verfahren hat den Vorteil, dass erst bei Erwärmung des Einkomponentenschaumstoffs das Material zu expandieren beginnt und sich die Schaumstoffmasse aus dem Einkomponentenschaumstoffs bildet, sobald die Kompressenkappe mit dem Einkomponentenschaumstoff an den Zahnstumpf, bzw. die Interdentalpapille gepresst wird.

Bei einem weiteren Verfahren zum Trocknen eines Zahnstumpfes und seiner Umgebung mittels einer Kompressenkappe sind nachfolgende Verfahrenschritte vorgesehen:
- Deponieren einer kühl gelagerten Komponente eines Einkomponentenschaumstoffs in einen Spender;
- Benetzen eines Zahnstumpfes mit dem Einkomponentenschaumstoff aus dem Spender;
- Anlegen der Kompressenkappe unter Einführen des Zahnstumpfes in den Hohlraum der Kompressenkappe;
- Anpressen des räumlich profilierten Öffnungsrandes der Kompressenkappe an die Interdentalpapille des Zahnstumpfes im Bereich der Gingiva-Tasche zum Abdichten des Sulcus;
- Bilden der saugfähigen Schaumstoffmasse aus dem Einkomponentenschaumstoff;
- Eindringen der Schaumstoffmasse in den Sulcus der Gingiva-Tasche;
- Aufsaugen der Körperflüssigkeiten durch die Schaumstoffmasse;
- Entfernen des Schaumstoffs von den getrockneten Oberflächen der Gingiva-Tasche und des Zahnstumpfes unter Abziehen der Kompressenkappe mit anhaftendem Schaumstoff.

Dieses Verfahren unterscheidet sich von dem vorhergehende dadurch, dass nicht die Kompressenkappe mit dem Einkomponentenschaumstoff befüllt wird, sondern der Zahnstumpf damit benetzt wird. Da die Ausbildung der Schaumstoffmasse bei Benetzung des Zahnstumpfes beginnt, muss die Kompressenkappe zeitnah auf den Zahnstumpf aufgesetzt werden.

Erfindungsgemäß kann der Schaumstoff in den vorstehenden Verfahren auch sowohl auf den Zahnstumpf aufgebracht als auch in die Kompressenkappe eingebracht werden.

Die Erfindung wird nun anhand der beigefügten Figuren näher erläutert.
- Figur 1: zeigt einen ersten schematischen Querschnitt durch eine Kompressenkappe einer ersten Ausführungsform der Erfindung;
- Figur 2: zeigt einen zweiten schematischen Querschnitt durch die Kompressenkappe der Figur 1 entlang der Schnittebene A - A;
- Figur 3: zeigt einen schematischen Querschnitt durch eine Kompressenkappe einer zweiten Ausführungsform der Erfindung;
- Figur 4: zeigt einen schematischen Querschnitt durch eine Kompressenkappe einer dritten Ausführungsform der Erfindung;
- Figur 5: zeigt einen schematischen Querschnitt durch eine Kompressenkappe einer vierten Ausführungsform der Erfindung;
- Figur 6: zeigt einen schematischen Querschnitt durch eine Kompressenkappe einer fünften Ausführungsform der Erfindung;

- Die Figuren 7 bis 10: zeigen Verfahrensschritte beim Trocknen eines Zahnstumpfes und seiner Umgebung;

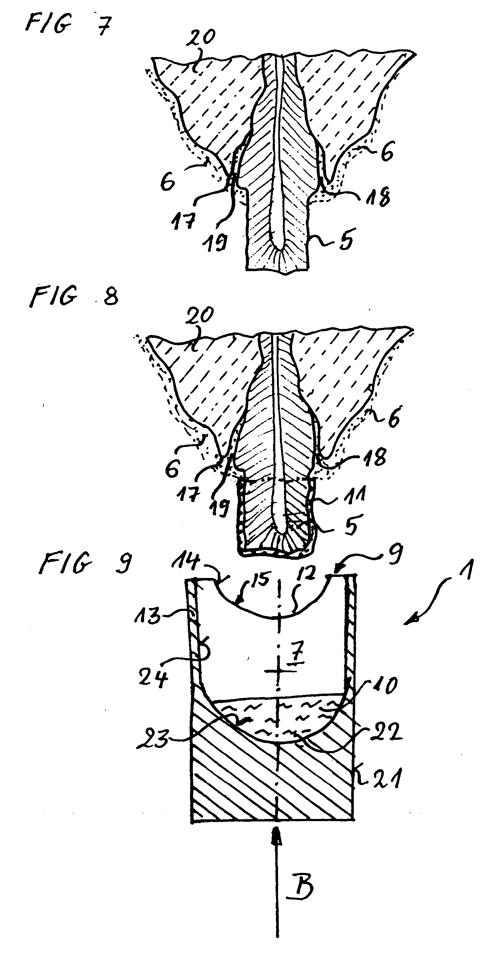

- Figur 7: zeigt einen schematischen Querschnitt durch einen Dentin-Zahnstumpf mit umgebendem Rand einer GingivaTasche;
- Figur 8: zeigt einen schematischen Querschnitt durch den Dentin-Zahnstumpf gemäß Figur 7 nach Benetzen des Zahnstumpfes mit einem Reagenz;
- Figur 9: zeigt einen schematischen Querschnitt durch eine Kompressenkappe nach Einfüllen eines Reagenz;

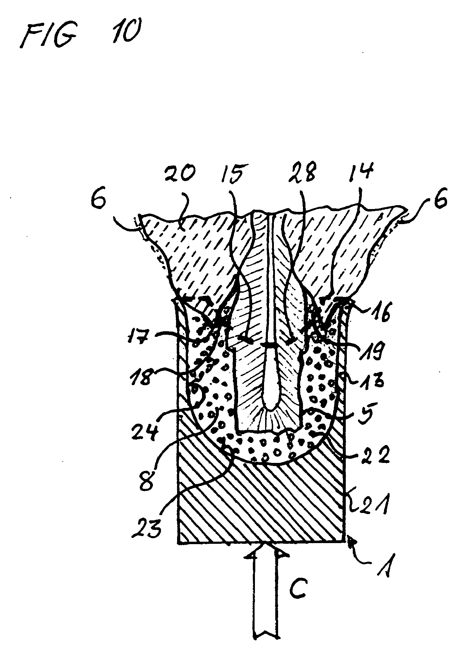

- Figur 10: zeigt einen schematischen Querschnitt durch die Kompressenkappe nach Bilden einer saugfähigen, den Zahnstumpf einbettenden expandierenden Schaumstoffmasse;

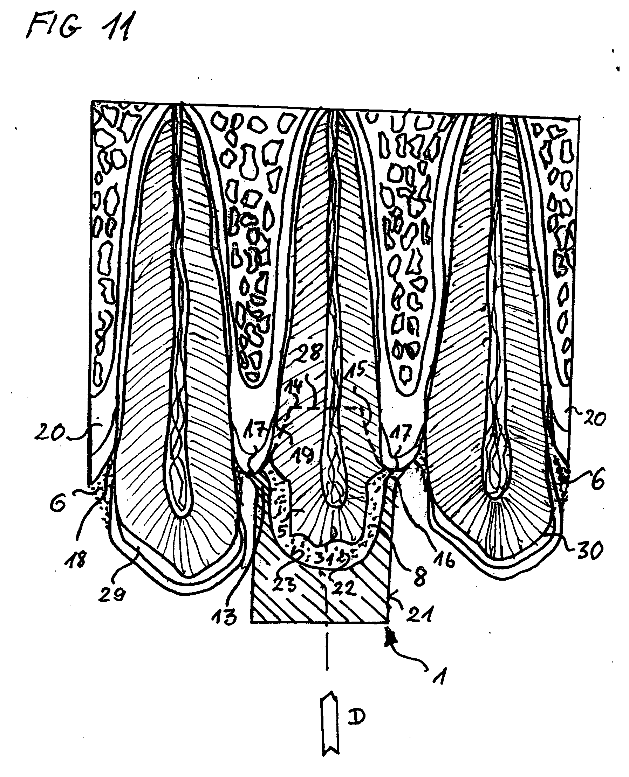

- Figur 11: zeigt einen schematischen Querschnitt durch drei benachbarte Zähne mit einem mittleren Zahnstumpf, der in eine saugfähige Schaumstoffmasse innerhalb einer Kompressenkappe eingebettet ist;
- Figur 12: zeigt eine Kappe gemäß dem Stand der Technik.

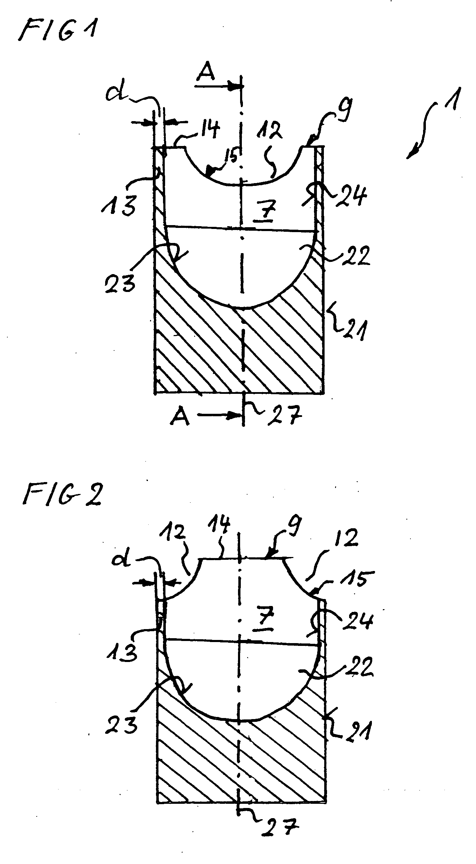

Figur 1 zeigt einen ersten schematischen Querschnitt durch eine Kompressenkappe 1 einer ersten Ausführungsform der Erfindung. Die Kompressenkappe 1 weist eine zylindrische Außenkontur 21 auf und ist aus einer Presswatte hergestellt. Die Kompressenkappe 1 weist einen Hohlraum 7 auf, der nach oben durch eine Öffnung 9 zugänglich ist. Der Hohlraum 7 ist von Wänden 13 umgeben, die eine zylindrische Innenkontur 24 aufweisen und im Bodenbereich 22 zu einer Halbkugelform übergehen. Ein Öffnungsrand 14 der Öffnung 9 weist ein räumliches Profil 15 auf, das mit zwei einander gegenüber liegenden Aussparungen 12 ausgestattet ist und sich aufgrund dieser Aussparungen 12 räumlich im angepressten Zustand der Kompressionskappe 1 an eine Interdentalpapille eines Zahnstumpfes im Bereich der Gingiva-Tasche dichtend anpassen kann.

Figur 2 zeigt einen zweiten schematischen Querschnitt durch die Kompressenkappe 1 der Figur 1 entlang der Schnittebene A - A. In diesem zweiten schematischen Querschnitt wird das Profil 15 des Öffnungsrandes 9 deutlich, wobei nun die in Figur 1 gezeigte kreisausschnittsförmige Aussparung 12 beidseitig der Mittellinie 27 der Kompressenkappe 1 angeordnet ist. Durch diese profilierte Konstruktion des Randes der Öffnung 9 wird erreicht, dass die Kompressenkappe 1 weiter über den Zahnstumpf geschoben werden kann, als das in der in Figur 12 gezeigten Kompressenkappe 4 gemäß dem Stand der Technik möglich ist. Dadurch wird auch erreicht, dass erstmalig die Kompressenkappe 1 nicht für ein Einpressen eines Refraktionsfadens einsetzbar ist, sondern eine Abdichtungsfunktion um einen zu trocknenden und abzudichtenden Zahnstumpf herum erfüllt. Komponenten mit gleichen Funktionen wie in Figur 1 werden mit gleichen Bezugszeichen gekennzeichnet und in den nachfolgenden Figuren nicht extra erörtert.

Figur 3 zeigt einen schematischen Querschnitt durch eine Kompressionskappe 2 einer zweiten Ausführungsform der Erfindung. Bei dieser zweiten Ausführungsform der Erfindung ist eine konische Innenwandkontur 26 für die Wände 13 des Hohlraumes 7 realisiert, die den Vorteil hat, dass die Wände im Bereich der Öffnung 9 eine minimale Wandstärke d aufweisen, die sich zum Bodenbereich 22 hin verstärkt und somit eine nach wie vor weiche Anpassung des Öffnungsrandes 14 an die Kontur der Interdentalpapille ermöglicht, während der untere Bereich verstärkt einen Anpressdruck auf die Interdentalpapille ausüben kann, ohne dass sich die Wände 13 im unteren Bereich und in unmittelbarer Nähe des Bodenbereichs 22 stark verformen.

Figur 4 zeigt einen schematischen Querschnitt durch eine Kompressenkappe 3 einer dritten Ausführungsform der Erfindung. In dieser Ausführungsform der Erfindung wird zum Bodenbereich hin die Wandstärke d durch eine parabolische Innenkontur 25 des Hohlraums 7 verstärkt.

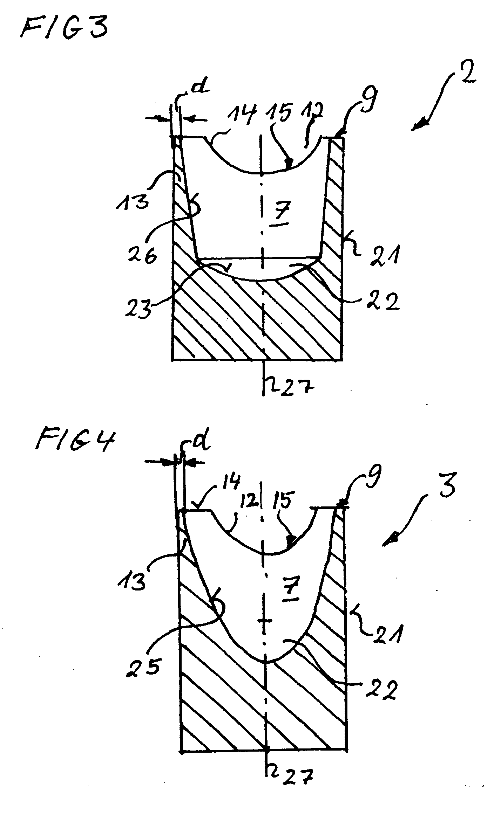

Figur 5 zeigt einen schematischen Querschnitt durch eine Kompressenkappe 34 einer vierten Ausführungsform der Erfindung. Komponenten mit gleichen Funktionen wie in den vorhergehenden Figuren werden mit gleichen Bezugszeichen gekennzeichnet und nicht extra erörtert. Die vierte Ausführungsform der Erfindung unterscheidet sich von den vorhergehenden dadurch, dass die Wände (13) eine gestufte Innenkontur mit Stufen 32 aufweisen, die dem Zahnstumpf angepasst sind.

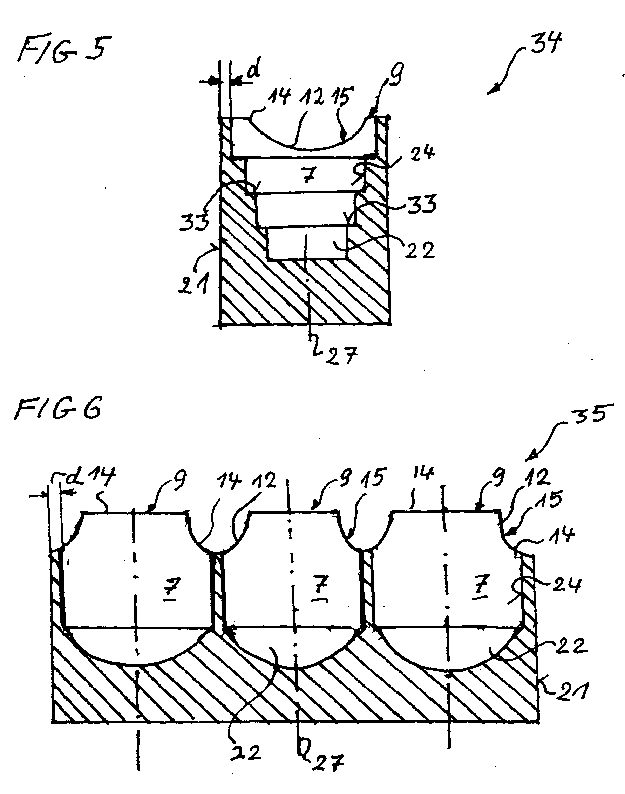

Figur 6 zeigt einen schematischen Querschnitt durch eine Kompressenkappe 35 einer fünften Ausführungsform der Erfindung. Komponenten mit gleichen Funktionen wie in den vorhergehenden Figuren werden mit gleichen Bezugszeichen gekennzeichnet und nicht extra erörtert. Die fünfte Ausführungsform der Erfindung unterscheidet sich von den vorhergehenden dadurch, dass die Kompressenkappe mehrere nebeneinanderliegende Hohlräume 7 für mehrere nebeneinander angeordnete Zahnstümpfe für eine gleichzeitige Trocknung von Zahnstümpfen aufweist. Eine derartige Kompressenkappe kann dahingehend erweitert werden, dass die Kompressenkappe eine Reihe nebeneinanderliegender Hohlräume 7 für eine Vielzahl zutrocknender Zahnstümpfe aufweist, wobei zwischen den Hohlräumen 7 Trennwände 32 angeordnet sind und wobei zwischen den Trennwänden Trennfugen angeordnet sind, um Einzelkompressenkappen oder auch mehrere Kompressenkappen von der Vielzahl der in Reihe angeordneten Hohlräume abzutrennen.

Die Figuren 7 bis 10 zeigen Verfahrensschritte beim Trocknen eines Zahnstumpfes 5 und seiner Umgebung. Dazu zeigt Figur 7 einen schematischen Querschnitt durch einen Dentin-Zahnstumpf 5 mit umgebendem Rand einer Gingiva-Tasche 18, die von einer Interdentalpapille 17 gebildet wird und einen Sulcus 19 zwischen dem Zahnstumpf 5 und der Gingiva 20 ausbildet. Dieser Spalt bzw. Sulcus 19, sowie die Oberfläche der Gingiva 20 sind üblicherweise mit Körperflüssigkeit 6 bedeckt, die Speichel und Blutspuren aufweisen kann. Mit den folgenden Darstellungen wird nun gezeigt, wie diese Bereiche um den Zahnstumpf 5 herum unter Zuhilfenahme der erfindungsgemäßen Kompressenkappe trockengelegt werden können.

Figur 8 zeigt dazu einen schematischen Querschnitt durch den Dentin-Zahnstumpf 5 gemäß Figur 7 nach Benetzen des Zahnstumpfes 5 mit einem Reagenz 11. Dieses Reagenz 11 kann auch lediglich am Ende des Zahnstumpfes 5 aufgetragen sein oder kann auch unabhängig vom Zahnstumpf 5 unmittelbar vor dem Zusammenfügen von Kompressenkappe 1, die in Figur 9 gezeigt wird, und dem Zahnstumpf 5 in den Hohlraum 7 der Kompressenkappe 1 der Figur 9 eingetropft werden, falls eine Benetzung des Zahnstumpfes 5 mit dem Reagenz 11 einer saugfähigen und expandierenden Schaumstoffmasse nicht möglich ist.

Figur 9 zeigt einen schematischen Querschnitt durch eine Kompressenkappe 1 nach Einfüllen eines Reagenz 10. Dieses Reagenz 10 ist ein weiteres Reagenz, das erforderlich ist, um in Kontakt mit dem Reagenz 11 eine Schaumstoff bildende Reaktion auszulösen. Deshalb ist dieses Reagenz 10 in dem Bodenbereich 22 mit halbkugelförmiger Innenkontur 23 der Kompressenkappe 1 in Gelform angeordnet. Die Gelform des Reagenz 10 verhindert, dass das Material der Kompressenkappe 1 das Reagenz 10 aunimmt. Nach dem Auffüllen des Bodenbereichs 22 mit dem Reagenz 10 kann nun die Kompressenkappe 1 in Pfeilrichtung B über den in Figur 8 gezeigten Zahnstumpf 5 geschoben werden, wobei sich der profilierte Rand 9 an die Form der Gingiva 20, welche den Zahnstumpf 5 umgibt, dichtend anpasst.

Figur 10 zeigt einen schematischen Querschnitt durch die Kompressenkappe 1 nach Bilden einer saugfähigen, den Zahnstumpf 5 einbettenden, expandierenden Schaumstoffmasse 8. Aufgrund der expandierenden Eigenschaft der Schaumstoffmasse 8, die durch Zusammenfügen der in den Figuren 8 und 9 gezeigten Reagenzien 10 und 11 entsteht, wird gleichzeitig der Sulcus 19 der Gingiva-Tasche 18 aufgeweitet und intensiv mit Schaumstoffmasse 8 befüllt, wobei die saugfähige Eigenschaft dieser Schaumstoffmasse 8 die Körperflüssigkeiten 6 aufnimmt. Dabei wird durch die erfindungsgemäße Ausbildung des Öffnungsrandes 14 eine Dichtung 16 geschaffen, die sich auf der Interdentalpapille 17 abstützt und die Gingiva-Tasche 18 mit dem Sulcus 19 abdichtet. Die gestrichelte Linie 28 stellt dabei den Verlauf des räumlichen Profils 15 des Öffnungsrandes 14 der Kompressenkappe 1 in ihrer ursprünglichen Form dar. Diese ursprüngliche Form wird durch das Andrücken in Richtung Pfeil C und durch das Ausbilden der Dichtung 16 leicht verzerrt.

Figur 11 zeigt einen schematischen Querschnitt durch drei benachbarte Zähne 29, 30 und 31, wobei der mittlere Zahn 31 einen Zahnstumpf 5 aufweist, der in eine die Oberflächen des Zahnstumpfes 5 trocknende und den Sulcus 19 aufweitende expandierende Schaumstoffmasse 8 innerhalb der Kompressenkappe 1 eingebettet ist. Die gestrichelte Linie 28 zeigt wiederum das ursprüngliche räumliche Profil 15 des Öffnungsrandes 14, der eine Dichtung 16 auf der Interdentalpapille 17 ausbildet. Während beim Einbetten des Zahnstumpfes 5 in die Schaumstoffmasse 8 die Kompressenkappe 1 in Pfeilrichtung D zur Bildung der Abdichtung 16 gepresst wird, kann nach Ausbilden der expandierenden und saugfähigen Schaumstoffmasse 8 diese Schaumstoffmasse 8 mit der aufgenommenen Körperflüssigkeit von dem Zahnstumpf 5 entfernt werden, sodass ein trockener Zahnstumpf zur Weiterbehandlung bspw. durch Abdrucknahme zur Verfügung steht. Die gestrichelte Linie 28 zeigt deutlich, dass durch den räumlich profilierten Öffnungsrand 14 die Gingiva 20 zum Gaumenbereich und zum Frontbereich hin weiter abgedeckt wird als in dem Bereich der Interdentalpapille 17 und somit ein Abdichteffekt rund um den Zahnstumpf 5 mit der erfindungsgemäßen Kompressenkappe 1 erreicht wird.

### Bezugszeichenliste

- 1: Kompressenkappe (1. Ausführung)
- 2: Kompressenkappe (2. Ausführung)
- 3: Kompressenkappe (3. Ausführung)
- 4: Kompressenkappe (gemäß Stand der Technik)
- 5: Zahnstumpf
- 6: Körperflüssigkeit
- 7: Hohlraum
- 8: Schaumstoffmasse
- 9: Öffnung des Hohlraums
- 10: erstes Reagenz
- 11: zweites Reagenz
- 12: Aussparung im Öffnungsrand
- 13: Wände des Hohlraums
- 14: Öffnungsrand
- 15: Profil des Öffnungsrandes
- 16: Dichtung
- 17: Interdentalpapille
- 18: Gingiva-Tasche
- 19: Sulcus
- 20: Gingiva
- 21: Außenkontur
- 22: Bodenbereich des Hohlraums
- 23: halbkugelförmige Innenkontur
- 24: zylindrische Innenkontur
- 25: parabolische Innenkontur
- 26: konische Innenkontur
- 27: Mittellinie
- 28: gestrichelte Linie
- 29: Zahn
- 30: Zahn
- 31: Zahn
- 32: Trennwand
- 33: Stufe
- 34: Kompressenkappe (4. Ausführung)
- 35: Kompressenkappe (5. Ausführung)

- d: Wandstärke der Wände des Hohlraums
- A-A: Schnittebene

## Patentansprüche

1. Kompressenkappe für Zahnstümpfe (5), wobei die Kompressenkappe (1) einen Hohlraum (7) zur Aufnahme einer einen Zahnstumpf (5) umgebenden saugfähigen Schaumstoffmasse (8) besitzt und wobei der Hohlraum (7) eine einseitige Öffnung (9) zur Aufnahme der Reagenzien (10, 11) für die Schaumstoffmasse (8) und zur Aufnahme des Zahnstumpfes (5) hat und wobei der Hohlraum (7) von Wänden (13) umgeben ist, deren Wandstärke (d) zur Öffnung hin vermindert ist und wobei der Öffnungsrand (14) über ein derartiges räumliches Profil (15) verfügt, dass der Öffnungsrand (14) eine Dichtung (16) im angepressten Zustand der Kompressenkappe (1) an die Interdentalpapille (17) eines Zahnstumpfes (5) im Bereich der Gingiva-Tasche (18) für den Sulcus (19) darstellt.

2. Kompressenkappe nach Anspruch 1,
**dadurch gekennzeichnet, dass** die Kompressenkappe ein elastomeres Material vorzugsweise aus Silikon aufweist.

3. Kompressenkappe nach Anspruch 1,
**dadurch gekennzeichnet, dass** die Kompressenkappe ein kompressibles Material, vorzugsweise eine für Körperflüssigkeiten (6) saugfähige gepresste Watte und/oder einen für Körperflüssigkeiten saugfähigen Filz und/oder einen für Körperflüssigkeiten saugfähigen Schaumstoff, aufweist.

4. Kompressenkappe nach Anspruch 1,
**dadurch gekennzeichnet, dass** die Kompressenkappe ein weichelastisches Material, das plastisch verformbar ist, aufweist.

5. Kompressenkappe nach einem der Ansprüche 1 bis 4,
**dadurch gekennzeichnet, dass** die Kompressenkappe (1) eine zylindrische Außenkontur (21) aufweist.

6. Kompressenkappe nach einem der Ansprüche 1 bis 4,
**dadurch gekennzeichnet**, die Kompressenkappe (1) einen elliptischen Querschnitt aufweist.

7. Kompressenkappe nach einem der Ansprüche 1 bis 4,
**dadurch gekennzeichnet, dass** die Kompressenkappe einen rechteckigen Querschnitt aufweist, und einen Außenmantel besitzt, der glatte Flächen aufweist.

8. Kompressenkappe nach einem der vorhergehenden Ansprüche,
**dadurch gekennzeichnet, dass** der Hohlraum (7) im Bodenbereich (22) eine halbkugelförmige Innenkontur (23) aufweist.

9. Kompressenkappe nach einem der vorhergehenden Ansprüche,
**dadurch gekennzeichnet, dass** die Wände (13) eine zylindrische Innenkontur (24) aufweisen.

10. Kompressenkappe nach einem der Ansprüche, 1 bis 8,
**dadurch gekennzeichnet, dass** die Wände (13) eine parabolische Innenkontur (25) aufweisen.

11. Kompressenkappe nach einem der Ansprüche, 1 bis 8,
**dadurch gekennzeichnet, dass** die Wände (13) eine konische Innenkontur (26) aufweisen.

12. Kompressenkappe nach einem der Ansprüche, 1 bis 8, **dadurch gekennzeichnet, dass** die Wände (13) eine elliptische Innenkontur aufweisen.

13. Kompressenkappe nach einem der vorhergehenden Ansprüche,
**dadurch gekennzeichnet, dass** die Wände (13) eine gestufte Innenkontur aufweisen.

14. Kompressenkappe nach einem der vorhergehenden Ansprüche,
**dadurch gekennzeichnet, dass** die Wandstärke (d) im Bereich der Öffnung (9) einer Zahnschmelzdicke entspricht.

15. Kompressenkappe nach einem der vorhergehenden Ansprüche,
**dadurch gekennzeichnet, dass** der Öffnungsrand (14) scheibenförmig ist und im Profil (15) zwei gegenüberliegende Aussparungen (12) aufweist, die sich bei Anpressdruck auf die Interdentalpapille (17) im Bereich einer Gingiva-Tasche (18) anschmiegen.

16. Kompressenkappe nach Anspruch 15, **dadurch gekennzeichnet, dass** die gegenüber liegenden Aussparungen (12) des Öffnungsrandes (14) als räumliches Profil (15) jeweils einen Kreisausschnitt aufweisen.

17. Kompressenkappe nach einem der vorhergehenden Ansprüche,
**dadurch gekennzeichnet, dass** die Kompressenkappe mehrere nebeneinanderliegende Hohlräume für mehrere nebeneinander angeordnete Zahnstümpfe für eine gleichzeitige Trocknung aufweist.

18. Kompressenkappe nach Anspruch 15, **dadurch gekennzeichnet, dass** die Kompressenkappe eine Reihe nebeneinanderliegender Hohlräume für eine Vielzahl zu trocknender Zahnstümpfe aufweist, wobei zwischen den Hohlräumen Trennwände angeordnet sind und wobei zwischen den Trennwänden Trennfugen angeordnet sind, um Einzelkompressen oder auch mehrere Kompressen von der Vielzahl der in Reihe angeordneten Hohlräume abzutrennen.

19. Verfahren zum Trocknen eines Zahnstumpfes (5) und seiner Umgebung mittels einer Kompressenkappe (1) gemäß einem der Ansprüche 1 bis 18, wobei das Verfahren folgende Verfahrenschritte aufweist,
- Deponieren eines ersten Reagenz (10) in einer ersten Kammer eines Mischers und Spenders;
- Deponieren eines zweiten Reagenz (11) in einer zweiten Kammer eines Mischers und Spenders;
- Mischen der beiden Reagenzien in einer Mischkammer des Mischers;
- Spenden einer vorgesehenen Menge der gemischten Reagenzien in eine Kompressenkappe und/oder auf einen Zahnstumpf;
- Anlegen der Kompressenkappe (1) unter Einführen des Zahnstumpfes (5) in den Hohlraum (7) der Kompressenkappe (1) ;
- Anpressen des räumlich profilierten Öffnungsrandes (14) der Kompressenkappe (1) an die Interdentalpapille (17) des Zahnstumpfes (5) im Bereich der Gingiva-Tasche (18) zum Abdichten des Sulcus (19);
- Bilden der saugfähigen Schaumstoffmasse (8) aus den gemischten Reagenzien (10, 11);
- Aufsaugen der Körperflüssigkeiten (6) durch die Schaumstoffmasse (8); und
- Entfernen des Schaumstoffs (8) unter Abziehen der Kompressenkappe (1) mit anhaftendem Schaumstoff (8).

20. Verfahren zum Trocknen eines Zahnstumpfes (5) und seiner Umgebung mittels einer Kompressenkappe (1) gemäß einem der Ansprüche 1 bis 18, wobei das Verfahren folgende Verfahrenschritte aufweist,
- Deponieren einer kühlgelagerten Komponente eines Einkomponentenschaumstoffs in einen Spender;
- Spenden einer vorgesehenen Menge des Einkomponentenschaumstoffs in eine Kompressenkappe und/oder auf einen Zahnstumpf;
- Anlegen der Kompressenkappe (1) unter Einführen des Zahnstumpfes (5) in den Hohlraum (7) der Kompressenkappe (1) ;
- Anpressen des räumlich profilierten Öffnungsrandes (14) der Kompressenkappe (1) an die Interdentalpapille (17) des Zahnstumpfes (5) im Bereich der Gingiva-Tasche (18) zum Abdichten des Sulcus (19);
- Bilden der saugfähigen Schaumstoffmasse (8) aus dem Einkomponentenschaumstoff;
- Aufsaugen der Körperflüssigkeiten (6) durch die Schaumstoffmasse (8); und
- Entfernen des Schaumstoffs (8) unter Abziehen der Kompressenkappe (1) mit anhaftendem Schaumstoff (8).
